# EUROPEAN PATENT SPECIFICATION

(11) **EP 2 593 789 B1**
(45) Date of publication and mention of the grant of the patent: **29.04.2015**
(21) Application number: 11754752.1
(22) Date of filing: 13.07.2011
(51) Int. Cl.: G01N 33/564

(54) **USE OF ANTI-PLAC1 PROTEIN ANTIBODIES AS BIOMARKERS OF INFERTILITY**
VERWENDUNG VON ANTI-PLAC1 PROTEIN ANTIKÖRPERN ALS BIOMARKER FÜR UNFRÜCHTBARKEIT
UTILISATION DES ANTICORPS CONTRE PLAC1 COMME BIOMARQUEURS D'INFERTILITÉ

(30) Priority: 14.07.2010 IT RM20100386
(43) Date of publication of application: 22.05.2013
(73) Proprietor: Universita 'Degli Studi Di Foggia, 71122 Foggia (IT)
(72) Inventor: LISO, Arcangelo, I-71100 Foggia (IT); MATTEO, Maria, I-71100 Foggia (IT)
(74) Representative: Gitto, Serena
(86) International application number: PCT/IT2011/000245
(87) International publication number: WO 2012/007981

(56) References cited:
- WO-A1-2010/065944
- DONG XUE-YUAN ET AL: "PLAC1 is a tumor-specific antigen capable of eliciting spontaneous antibody responses in human cancer patients", INTERNATIONAL JOURNAL OF CANCER, JOHN WILEY & SONS, INC, UNITED STATES, SWITZERLAND, GERMANY, vol. 122, no. 9, 1 May 2008 (2008-05-01), pages 2038-2043, XP009102971, ISSN: 0020-7136, DOI: DOI:10.1002/IJC.23341
- SILVA JR W A ET AL: "PLAC1, a trophoblast-specific cell surface protein, is expressed in a range of human tumors and elicits spontaneous antibody responses", CANCER IMMUNITY 20071106 CH, vol. 7, 6 November 2007 (2007-11-06), XP002615924, ISSN: 1424-9634
- KOSLOWSKI MICHAEL ET AL: "A placenta-specific gene ectopically activated in many human cancers is essentially involved in malignant cell processes", CANCER RESEARCH, AMERICAN ASSOCIATION FOR CANCER RESEARCH, US, vol. 67, no. 19, 1 October 2007 (2007-10-01), pages 9528-9534, XP002471063, ISSN: 0008-5472, DOI: 10.1158/0008-5472.CAN-07-1350
- MICHAEL FANT ET AL: "The PLAC1 protein localizes to membranous compartments in the apical region of the syncytiotrophoblast", MOLECULAR REPRODUCTION AND DEVELOPMENT, vol. 74, no. 7, 1 July 2007 (2007-07-01), pages 922-929, XP55013187, ISSN: 1040-452X, DOI: 10.1002/mrd.20673
- MICHAEL FANT ET AL: "PLAC1 (Placenta-specific 1): a novel, X-linked gene with roles in reproductive and cancer biology", PRENATAL DIAGNOSIS, 1 January 2010 (2010-01-01), pages N/A-N/A, XP55013261, ISSN: 0197-3851, DOI: 10.1002/pd.2506

## Description

The present invention concerns the use of antibodies against PLAC1 protein as biomarkers of infertility. A diagnostic kit for the detection of the immune response against PLAC1 and use of PLAC1 protein in therapeutic and contraceptive fields is disclosed.

In particular, disclosed is the use of antibodies against PLAC1 protein as biomarkers of infertility, diagnostic kit for the detection of the immune response against PLAC1 and use of PLAC1 protein in tolerogenic or immunogenic form for infertility therapy or as contraceptive or means of post-coital interception, respectively.

Sterility is a constantly increasing pathology with an incidence from 3.5% in 1965 to 9.2% in 1982 (Bulletin WHO 2007; 85,733-820) and presently in Italy involving 10-15% fertile couples (i.e. 50.000-70.000 couple according to 2000 Istat data).

Among various sterility reasons, idiopathic sterility still covers a substantial percentage (approximately 15%) of carried out diagnoses. Just in this category in fact it does not exist, today, any known cause for good quality repeated deficit embryo implant, transferred into young patient uteri and without apparent sterility cause. Given the fact that in the field of idiopathic sterility no pathogenic biological mechanism has been detected and consequently diagnostic tests are not available, it remains an exclusion diagnosis (and that it is to be placed after the exclusion of already known causes). Finding a biomarker and pathogenic mechanism within this category represents a remarkable development step from the diagnostic and potentially therapeutic point of view.

In the light of above it is therefore apparent the need to provide for a diagnostic method and therapy for idiopathic sterility and embryo implant deficit in other origin sterility cases.

During the last few years a series of placenta-specific genes have been identified (Annu Rev Cell Dev Biol. 2008;24:159-81) of fundamental importance for the normal development of the placental functions, even if, up to now, the number of actually and exclusively placenta specific identified genes is very little.

PLAC1 (PLACenta-specific 1) is a trophoblast specific gene located on a locus of X chromosome, of remarkable importance for the development of placenta (Mol Reprod Dev. 2007; 74: 922-9). PLAC1 encodes for a surface protein highly expressed in placenta. In particular, PLAC1 protein is localized at the level of intracellular membranous compartment in apical region of the syncytiotrophoblast and it is expressed on membrane surface of placenta microvilli (Mol Reprod Dev. 2007; 74: 922-9). This protein has been previously identified also in patients suffering from hepatic cancer (HCC) (Int J Cancer. 2008; 122: 2038-43). In a percentage of these subjects it has been also demonstrated the presence of anti-PLAC1 antibody, demonstrating the immunogenic properties of the protein and ability thereof to induce an antibody response in such patient subset (Cancer Immunity 2007; 6:7 - 18).

Up to now in obstetric field, PLAC1 mRNA presence has been detected in pregnancies characterized by placenta damage, in preeclamptic patients (Prenat Diagn. 2007; 27: 772-7), but there are not literature data about the presence of anti-PLAC1 and/or PLAC1 mRNA data in infertile patients. In addition, Fant et al. (Fant et al., Prenat Diagn 2010;30:497-502) describes that PLAC1 elicits an antibody response in some cancer patients which promotes their survival. It also discusses a potential role for PLAC1 as a biomarker predictive of specific pregnancy complications, such as preeclampsia.

The authors of the present invention now have found, for the first time, the presence of antibodies against PLAC1 protein in patients affected from idiopathic sterility characterized by repeated failures of embryo implant after IVF-ET cycles. The level of anti PLAC1 antibodies is remarkably higher than control fertile patients. PLAC1 is a recently detected, placenta-specific gene which is bound to the long arm of X chromosome. (Genomics. 2000 15; 68: 305-12).

Previous in situ hybridization studies with antisense mRNA during murine embryogenesis process proved the expression of PLAC1 gene from 7,5 to 14,5 days after fecundation, resulting in the opinion that PLAC1 is a marker of placenta development with a key role in the development of the maternal-foetal interface (Genomics. 2000 15; 68: 305-12). Moreover PLAC1 has been detected to be expressed during all the gestation from 8 to 41 week and it is constantly trophoblast-specific. (Mol Reprod Dev. 2002; 63: 430-6). Being an immunogenic protein (Cancer Immunity 2007; 6:7 - 18) localized on the apical surface of the placenta trophoblast microvilli (Mol Reprod Dev. 2007; 74: 922-9) the authors assumed that the presence of anti-PLAC1 antibodies could be associated to infertility conditions preventing the physiological process of embryo implant. Thus it has been studied a subset of patients affected from idiopathic type sterility (Hum Reprod 1995; 10: 1246-71) and repeated failures of embryo implant after IVF-ET cycles wherein the presence of higher levels of anti PLAC1 antibodies could be associated to embryo implant problems. Results supports above said hypothesis. In fact the statistical analysis demonstrates that in sterile patients the concentration of anti PLAC1 antibodies is remarkably higher than fertile controls.

It is interesting to emphasize the clinical data of follow-up carried out during the successive year on recruited sterile patients. In fact out of 5 patients with greater levels of anti-PLAC1 antibodies none succeeded to be pregnant during the successive months, both spontaneously or after repeated IVF-ET cycles and one was affected from 2 abortions and a biochemical pregnancy (i.e.: pregnancy demonstrated only by positive BHCG test in the absence of gestation bag evidence). Instead out of 17 sterile patients with not significant levels of anti PLAC1 antibodies, 6 (35%) succeeded to obtain a developed pregnancy in the course of the follow-up.

Therefore such data demonstrate the presence of antibodies against placenta-specific PLAC1 antigen in patients affected from idiopathic type sterility with remarkably higher titres than healthy fertile controls. Clinic follow-up data suggest that antibody response occurs in a subset of infertile patients and determines the reproduction prognosis thereof. It cannot also be excluded that an anti-body response against PLAC1 during pregnancy can be elicited resulting in a placenta damage and can be correlated to pathologies such as preeclampsia or foetal growth defects from placenta insufficiency. In this context the test could have to be carried out also for monitoring of the pregnancy.

It has been moreover demonstrated the possibility to develop an antibody response against PLAC1 as a result of the administration of protein peptide sequences (antigenic epitopes). It is therefore reasonable to argue that PLAC1 can be used in order immunity (or tolerance) for contraceptive end or infertility therapy to be actively induced.

It is therefore a specific object of the present invention the use of antibodies against PLAC1 protein as biomarkers of infertility, for example idiopathic type, in women and animal females expressing the protein.

Examples of animal females expressing PLAC1 protein are primate (*Pan troglodytes*)*,* bovine (*Bos taurus*), equine (*Equus caballus*), swine (sus scrofa), mouse (*Mus musculus*), rat (*Rattus norvegicus*).

The present invention further concerns a method for *in vitro* infertility diagnosis, for example idiopathic type, in women and animal females expressing said protein by detection of anti PLAC1 immune response, said immune response being selected from cell-mediated or antibody response. Animal females expressing PLAC1 protein are for example primate (*Pan troglodytes*), bovine (*Bos taurus*), equine (*Equus caballus*), swine (sus scrofa), mouse (*Mus musculus*), rat (*Rattus norvegicus*).

A kit for the *in vitro* infertility diagnosis, for example idiopathic type, in women and animal females expressing PLAC1 protein can be used, said kit comprising or being consisting of peptide from aminoacid 61 to aminoacid 128 of human (or murine) PLAC1 or from aminoacid 126 to aminoacid 155 of human PLAC1 or at least a portion of said peptide as for example, CPPNHVQPHAYQFTYRVTE (SEQ ID NO: 5), CGIRAKAVSQDMVIYST (SEQ ID NO: 6), EIHYSSKGTPSKFVIPVSC (SEQ ID NO: 7), AAPQKSPWLTKPC (SEQ ID NO: 8), KPCSMRVASKSR (SEQ ID NO: 9), KSRATAQKDEKC (SEQ ID NO: 10), EKCYEVFSLSQS (SEQ ID NO: 11).

Animal females expressing PLAC1 protein are for example primate (*Pan troglodytes*)*,* bovine (*Bos taurus*), equine (*Equus caballus*), swine (sus scrofa), mouse (*Mus musculus*), rat (*Rattus norvegicus*).

The above mentioned kit can be used for example for/in ELISA test or method measuring cell-mediated response against PLAC1.

ELISA test for the determination of anti-PLAC1 antibody titre should be carried out on all the patients affected from idiopathic type sterility before IVF-ET cycle administration, aiming to predict repeated embryo implant deficits. However, it is noteworthy to emphasize that the anti-PLAC1 antibody presence would have to be tested in all the other infertile patient categories, (comprising also repeated abortion affected patients) in addition to already suggested procedures according to infertility state base diagnosis (Van den Eede B. Investigation and treatment of infertile couples: ESHRE guidelines for good clinical and laboratory practice. Hum Reprod 1995;10:1246-71). The test could have to be carried out also in the pregnancy monitoring in order placenta damage to be excluded. Given PLAC1 immunogenicity, it is reasonable that T lymphocyte clones are suitable to recognize the antigen. Therefore tests measuring the cell-mediated response in any form, against PLAC1, can be proved to be equally important for diagnosis of reproduction pathologies.

The recombinant PLAC1 protein, at least a portion thereof or a PLAC1 encoding nucleotide sequence or a portion thereof in a vector could be used for contraceptive treatment or post-coital interception. Said portion can be selected from peptide from aminoacid 61 to aminoacid 128 of human (or murine) PLAC1 or from aminoacid 126 to aminoacid 155 of human PLAC1 or at least a peptide selected from CPPNHVQPHAYQFTYRVTE (SEQ ID NO: 5), CGIRAKAVSQDMVIYST (SEQ ID NO:6), EIHYSSKGTPSKFVIPVSC (SEQ ID NO:7), AAPQKSPWLTKPC (SEQ ID NO:8), KPCSMRVASKSR (SEQ ID NO:9), KSRATAQKDEKC (SEQ ID NO:10), EKCYEVFSLSQS (SEQ ID NO:11)

The induction of an important anti PLAC1 response by inoculation of recombinant protein, portions thereof, or DNA encoding therefor in any vector can be used as means of post-coital interception and possible contraception by preventing blastocyst nesting. This could be carried out as an alternative to existing methods based on administration of ad hoc drugs, for example those based on progestins and/or estrogens.

In addition, PLAC1 protein could be used for treatment of infertility. Particularly, PLAC1 protein or at least a portion thereof can be administered in a tolerogenic form. In fact, for subjects at risk of immune response against PLAC1 development, due to genetic background or other causes, immunological tolerance, for example by means of administration in tolerogenic form of antigen or analogous thereof, could be induced resulting in induction of regulatory T cells or anergy of previously developed immune cells (Nat Rev Immunol. 2008 December; 8:970 - 976). Alternatively, antibodies anti-antibodies (i.e. anti-idiotype antibodies against the variable regions of anti-PLAC1 antibodies) can be generated in the treatment of infertility. Moreover, PLAC1 or portions thereof can be used for plasma selective removal of anti-PLAC1 antibodies (or portions thereof) by applying the immunoabsorption principle in the treatment of infertility. Said portion, to be administered aiming to induce tolerance, can be selected from the peptide from aminoacid 61 to aminoacid 128 of PLAC1 or from aminoacid 126 to aminoacid 155 of PLAC1 or at least a peptide selected from CPPNHVQPHAYQFTYRVTE (SEQ ID NO: 5), CGIRAKAVSQDMVIYST (SEQ ID NO: 6), EIHYSSKGTPSKFVIPVSC (SEQ ID NO: 7), AAPQKSPWLTKPC (SEQ ID NO: 8), KPCSMRVASKSR (SEQ ID NO: 9), KSRATAQKDEKC (SEQ ID NO: 10), EKCYEVFSLSQS (SEQ ID NO: 11).

PLAC1 protein or at least a portion thereof or PLAC1 encoding nucleotide sequence or a portion thereof or anti-antibody against PLAC1 can be applied, according to above mentioned uses, for both in medical and veterinary field. In fact, because PLAC1 encoding gene occurs in many other animal species (Annu. Rev. Cell Dev. Biol. 2008. 24:159-81), the same diagnostic and therapeutic applications, as described for human, can be extended to veterinary field.

The present invention now will be described by an illustrative, but not limitative way, according to preferred embodiments thereof, with particular reference to enclosed drawings, wherein:
Figure 1 shows gel-electrophoresis (SDS-PAGE) analysis results after purification of 1 ml of product on Ni⁺ column 1: lysate; 2: penetration; 3-13: various elution fractions.
Figure 2 shows the analysis results as in figure 1 but carried out on a 3 ml product volume.
Figure 3 shows gel-electrophoresis (SDS-PAGE) analysis results about the isolation of inclusion bodies 1-6: suspension after washing; 7: purified inclusion bodies.
Figure 4 shows gel-electrophoresis (SDS-PAGE) analysis results on eluted fractions (3-13) after elution on Ni⁺ column.
Figure 5 shows the results of analysis on recombinant protein after refolding.
Figure 6 shows the optical density distribution in individual cases and analyzed controls (dilution 1:101).
Figure 7 shows average values of optical density and relative standard deviation obtained by ELISA assays at various dilutions in the evaluation for the presence of antibodies against SEQ ID NO: 5-8 peptides after mouse immunization.

### Example 1: Study of immune response against PLAC1 protein in patients with idiopathic sterility

### MATERIALS AND METHODS

### Patients

Experimental sera have been obtained from 22 young patients suffering from idiopathic type sterility, (sterile group) with an history of embryo implant repeated failures in presence of IVF-ET cycles. Control samples have been obtained from 83 fertile women (control group). Among controls, for 73 control women the withdrawal has been carried out within 72 hours after delivery, while other 10 samples have been obtained from women at our Institute for gynaecological control visit. For both groups the exclusion criteria have been as following: ages > 40 years, presence of systemic pathologies and/or more than two abortions during first trimester, pharmacological or hormonal therapies in course or finished within less than 6 months with respect to control visit.

The informed written consent has been obtained from all the patients at recruitment for the study also approved by the local ethical committee.

The clinical characteristics of recruited patients are reported in Tab. 1.

All the infertile women have been subjected to suggested diagnostic examinations for evaluation of the infertility state: (Rev Obstet Gynecol 2008; 1: 69-76): analysis of partner seminal fluid, test for ovulation evaluation (luteal phase progesterone dosage, LH kit), evaluation of ovarian reserve, transvaginal echography, and isterosalpingography. The diagnosis of idiopathic sterility is carried out by exclusion, being excluded anomalies for above mentioned diagnoses (Rev Obstet Gynecol 2008; 1: 69-76, Hum Reprod 1995; 10: 1246-71).

The diagnosis for repeated and unexplained embryo implant deficit has been carried out after the failure of three IVF-ET cycles with transfer of good quality not frozen embryos in patients with good ovarian reserve.

In Table 1 clinical characteristics of recruited patients in the study are reported.

Data are expressed as average ± SD

**Table 1**

| | Sterile group n 22 | Control group n 82 |
|---|---|---|
| **Age** | 36,1 + 3,2 | *33,9*± *3,3* |
| **N ° of abortions First trimester** | 0,2 + 0,6 | *1,1 ±0,4* |
| **N°of evolutive pregnancies** | *0 + 0* | *2,2 ± 0,8* |
| **N° of IVF-ET attemps** | *4+ 0,5* | *0 ± 0* |

### METHODS

### Production of PLAC1-10His in Escherichia strains

### Cloning of PLAC1gene

Gene region from 67 to 636 nucleotide of encoding sequence (NM_021796) and corresponding to 23-212 aminoacids of PLAC1 protein has been cloned in p2N (Primm, Milan Italy) vector wherein 10 His TAG at N-terminus end is present. Cloned region codifies for the protein with a molecular weight of approximately 21 KDa.

The official site describing both gene and protein is at website http://www.ncbi.nlm.nih.gov/gene/10761

The protein is indicated according to the following way:
placenta-specific protein 1 precursor [Homo sapiens] NCBI Reference Sequence: NP_068568.1
>gi|11496283|ref|NP_068568.1| placenta-specific protein 1 precursor [Homo sapiens]

Website: http://www.ncbi.nlm.nih.gov/protein/11496283?report=fasta

The gene fragment to be cloned has been obtained by PCR amplification of cDNA extracted from placenta and bone marrow. Oligonucleotides used for PCR reaction are named FORlic and REVlic and sequences thereof are the following ones:
FORlic
   5'- caccaccacggcgtcCAAAGTCCAATGACTGTGCTGTGC -3' (SEQ ID NO: 2)
REVlic
   5'-cgagcgaaggcgtcagattaTCACATGGACCCAATCATATCATCTGTGTG-3' (SEQ ID NO: 3)

The cloning has been carried out in p2N vector using LIC technique (Ligation Indipendent Cloning) (Nucleic Acids Res. 18, 6069-6074; Biotechniques. 4, 515-518).

### Expression and purification of PLAC1 protein

### Pilot expression of PLAC1 in E. coli bacterium BL21 (DE3) strain - experiment 1

BL21 (DE3) host strain has been used for the IPTG-induced pilot expression of PLAC1 protein, overnight to 25°C and by purification using "Ni+ Sepharose High Performance" column.

Buffer:
a) Lysis Buffer: 50 mM Tris, 300 mM NaCl, 5 mM imidazole, 5 mM β-ME, 1% Triton-X-100, 1 mM PMSF, pH=8.0
b) Buffer A: 50 mM Tris, 300 mM NaCl, 5 mM imidazole, 5 mM β-ME, pH=8.0
c) Buffer B: 50 mM Tris, 300 mM NaCl, 500 mM imidazole, 5 mM β-ME, pH=8.0

Procedure:
a) 3µL of BL21 (DE3) /PLAC1 strain have been inoculated in 3ml of 2YT medium (two tubes). Then Amp and Kan have been added at a final concentration of 100µg/mL and 20µg/mL, respectively
b) 100µL of culture have been transferred in 1L of 2YT medium (5 flasks) over the night, and Amp and Kan added at a final concentration of 100µg/mL and 20µg/mL, respectively, centrifuged at 25°C 220rpm, in such way that culture reaches an OR₆₀₀ value of 1.0. The protein expression has been induced by addition of IPTG at final concentration of 0.5 mM. The incubation has been carried out at 25°C, 220rpm over the night.
c) the cells have been harvested and wet weighted, then the pellet resuspended in the lysis buffer (1:10), and sonicated for 20 min on ice, with interval impulses. The cells have been centrifuged for 30 min, 16000 x g, at 4°C. The supernatant has been collected and prepared for Ni⁺ NTA purification.
d) lysate has been eluted through the previously buffer A equilibrated column, flow rate 1mL/min; then eluted with buffer B. Cell fractions have been collected till the effluent absorbance reached the base value.

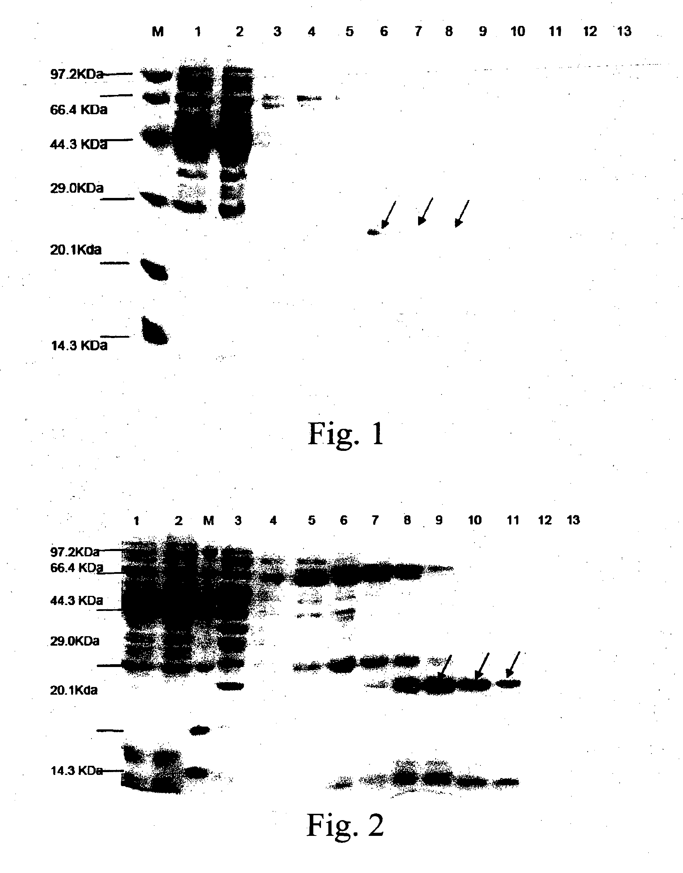

Each cell fraction has been analyzed by SDS-PAGE as shown in Figures 1 and 2.

### Conclusion

PLAC1 protein can be purified using His⁺ column and the purity thereof can reach 85%. We have collected number 6, 7, 8 tubes of Fig. 1 and # 9, 10, 11 tubes of Figure 2. However, it has been demonstrated that nor the purity nor the yield were according to our expectations

### Expression of PLAC1 in E. coli bacterium BL21 (DE3) strain-experiment 2.

Based on already described experiments for PLAC1 pilot expression, we used BL21 (DE3) bacterial strain. The protein expression has been induced over the night at 37°C. The target protein has been expressed in inclusion bodies and successively we have purified the inclusion bodies according to standard procedure.

### Results:

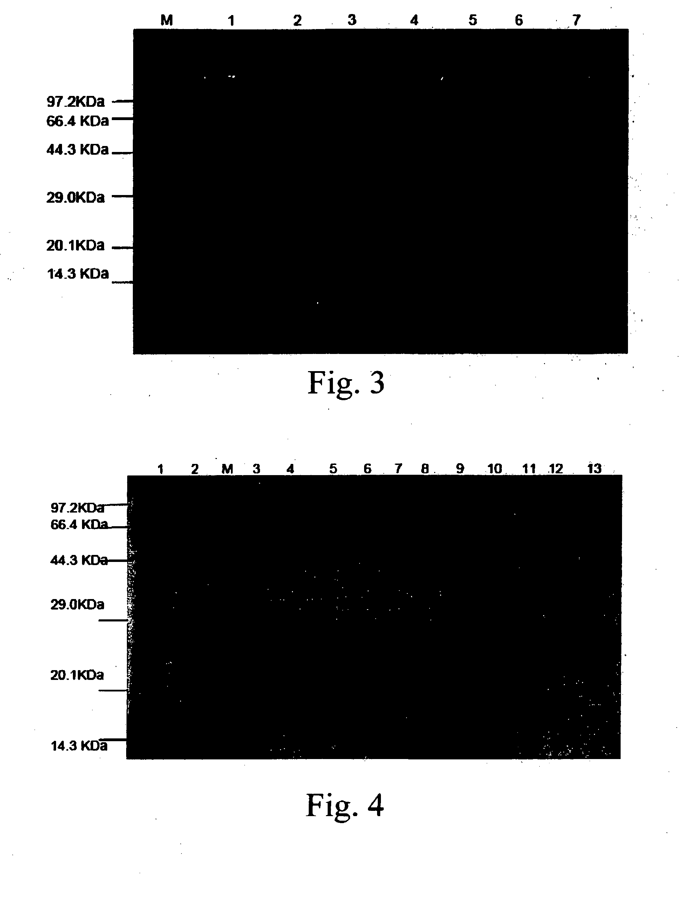

We have carried out the isolation of the inclusion bodies and washings: the results are reported in figure 3.

We have extracted the inclusion bodies (standard method).

The column has been loaded with Ni⁺ High Performance medium and buffer A equilibrated. Extracted inclusion bodies have been eluted through the column at a flow rate of 1 mL/min and then eluted with buffer B. The cell fractions have been collected till the effluent absorbance approached the base value.(4).

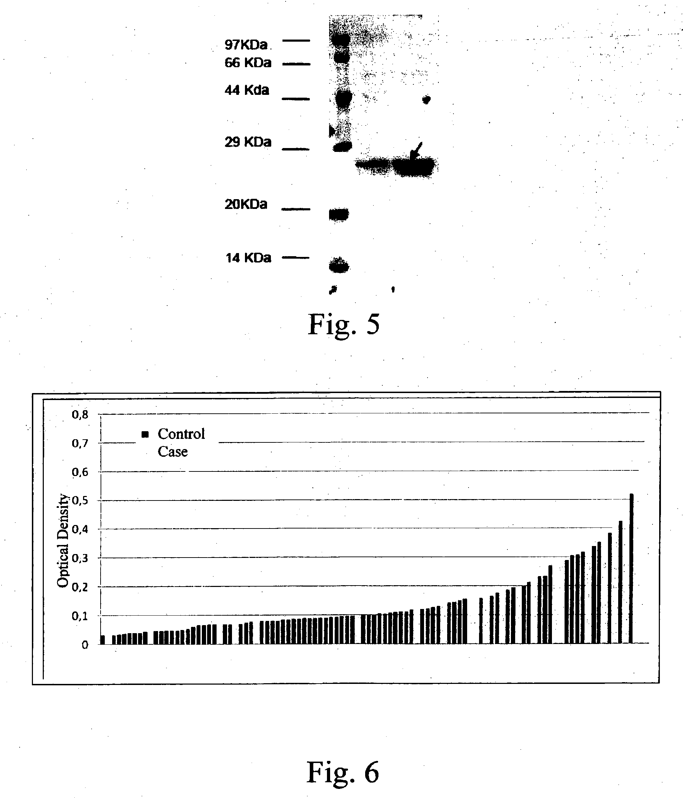

PLAC1 protein suitable for ELISA kit to be carried out has been obtained. The purity and yield were acceptable (figure 5).

### Setting of ELISA test for measurement of anti-PLAC1 antibody response.

### Principle:

ELISA Indirect method for detection of anti PLAC1 antibodies (IgG) in human serum.

### Protocol:

Setting with PLAC1: the antigen has been diluted in CB buffer at a final concentration of 16 µg/mL PVC plate wells have been coated with 100 µL of antigen. After the plate has been covered with plastic adhesive, the incubation has been carried out for 2 hours at 37°C.
a) The coating solution has been discarded and the plate washed 2 times with PBST.
b) Block: protein binding sites have been blocked by the addition of 150µL 10% FBS-PBS, incubated for 2 hours at 37°C.
c) Sample preparation: 1:101 dilution with 5% FBS.
d) Samples incubation: 100µL of each diluted sample have been added to each well and incubated for 1 hour at 37°C.
e) 5 washings of plate with PBST.
f) To each well 100µL of human HRP-conjugated anti-IgG have been added and incubated for 30 minutes at 37°C.
g) 5 washings of the plate with PBST.
h) Detection: 100µL of TMB substrate solution have been delivered to each well using a multichannel pipette. Incubated for 15 minutes at 37°C.
i) After sufficient colour development 50µL of blocking solution have been added to each well.
j) Absorbance reading (optical density) for each well with ELISA Plate reader.

It is noteworthy to point out that in these experiments the concentration of antigen has been optimized resulting in an optimal amount of 1,6 mg/well. Moreover it has been established that the optimal coating temperature is 37°C/2 hours and that no signal occurred when PLAC1 was incubated over the night at 4°C. The instability condition of PLAC1 protein can be one of possible causes.

*Reproducibility validation.*
a) We have produced a kit lot on 5 March and another one on 8 April. We have chosen 11 samples of which 9 negative controls and 2 patients clearly positive for antibody presence at high titre, then we have carried out kit reproducibility tests.

Conclusions: 100 % reproducibility coincidence rate. We set out a stabilized ELISA kit and the same is ready for the production.

**Table 2 is summarizing for reproducibility validation.**

| *N.* | ID | *Sample* | *Tested on 05*/*03*/*2010* | | *Tested* on *08*/*04*/*2010* | |
|---|---|---|---|---|---|---|
| | | | OD | | OD | |
| 1 | 1C | Control | 0.095 | | 0.074 | |
| 2 | 2C | Control | 0.033 | | 0.043 | |
| 3 | 3C | Control | 0.104 | | 0.098 | |
| 4 | 4C | Control | 0.144 | | 0.114 | |
| 5 | 5C | Control | 0.065 | | 0.106 | |
| 6 | 6C | Control | 0.090 | | 0.175 | |
| 7 | 7C | Control | 0.107 | | 0.076 | |
| 8 | 8C | Control | 0.426 | | 0.446 | |
| 9 | 9C | Control | 0.067 | | 0.025 | |
| 10 | 10C | Control | 0.068 | | 0.095 | |
| 11 | 31 | Patient | 0.612 | | 0.909 | |

### Specifications on PLAC1-Ab ELISA test (indirect).

### MATERIAL OCCURRING IN PRODUCED KIT

1. PLAC1 coated microwells- (12 x 8).
2. Washing buffer (20 x) - A 30 ml bottle containing saline concentrated buffer 20X (pH 7,2-7,6) with Tween 20 and preservative agent (0,1% ProclinTM). At low temperatures crystallization can occur. In order to maintain a clear coloration storage the solution at 37°C. Stir well. Dilute a part of washing buffer with 19 distilled water parts. Diluted buffer can be stored for a week at 2-25°C.
3. Diluting agent - A 50 ml bottle. Ready to be used. PBS (pH 7,2-7,6) with preservative (0.1% ProclinTM) and 5% FBS. Stable at 2-8 °C till the expiration.
4. HRP-conjugated anti-human IgG - A 13 ml bottle. Ready to be used. Goat-peroxidase conjugated human anti IgG with preservative (0.1 % ProclinTM) and protein stabilizing agents. Stable at 2-8 °C till the expiration.
5. A substrate - A 8mL bottle. Ready to be used. It contains hydrogen peroxide in citric acid-citrate buffer (pH 3,5-3,8). Stable at 2-8 °C till the expiration.
6. B substrate - A 8mL bottle. Ready to be used. It contains 3,3', 5,5" - tetramethylbenzidine and stable at 2-8°C till the expiration.
7. Positive control - A red stopper little bottle, 500 µL of diluted human serum (contains preservative and stabilizing agents). Stable at 2-8°C till the expiration.
8. Negative control - A 1 ml green stopper little bottle of human serum (contains preservative and stabilizing agents). Stable at 2-8°C till the expiration.
9. Blocking Solution - A 8 ml bottle. Ready to be used. 2M phosphoric acid. Stable at 2-25 ºC till the expiration.

Proclin™ 300 is a registered trademark product from *Rohm and Haas Company.* (Xn - dangerous) Control precautions and dispenser safety: concentration of sodium azide in these components is classified as harmful and according to the following risk sentences (R22, R32) "Injurious by ingestion" and "At contact with acids very toxic gas is released."

### Material requested but NOT PROVIDED WITHIN PRODUCED KIT

1. Adjustable accurate micropipettor equipped with disposable pipettes
2. Tips (5-1.000µ)
3. Deionized water
4. Microplate washing system
5. Microplate reader with 450 nm filter
6. Timer
7. Graduated cylinder
8. Flasks
9. Test-tube or microtitration plate for serum dilution

### COLLECTION AND PREPARATION

Blood obtained by means of venous bleeding would clot at room temp. (20-25 º C) and then centrifuged according to Clinical and Laboratory Standards (CLSI). The serum must be separated as soon as possible and stored in refrigerator (2-8°C) or frozen (≤ -20°C), if not tested within two days. Use of jaundiced or haemolysis, hyperlipidemia or microbial growth showing sera is recommended.

### DETAILED ELISA TEST PROCEDURE

All the reagents must be conserved at room temp. (20-25 ºC) before beginning the test. If the test is carried out not according to time and temperature range recommendations not valid results can be obtained. Tests carried out not according to suggested time and temperature range must be repeated.
1. Remove requested number of wells from the envelop and insert the same within strip-carrier. Five microwells are requested for negative control, positive control in triplicate. Make sure that remaining unused microwells are tightly closed within the aluminium envelope.
2. Use adequate test-tube or microtitrer plate, dilute the patient sample. To 5 µL of serum add 500 µL of sample diluting agent. Stir well.
3. Transfer using a pipette 100 ul of diluted patient sample, controls and dispensers in respective wells. Cover the plate and incubate for 60 ± 5 37 minutes at 37º C ± 1ºC.
4. Wash five (5) times with diluted washing buffer (refer to washing procedure).
5. Transfer using a pipette 100 ul of HRP-conjugated anti-human IgG in each well. Cover the plate and incubate for 30 ± 2 minutes at 37ºC ± 1ºC.
6. Wash five (5) times with diluted washing buffer (refer to washing procedure)
7. Transfer using a pipette 50 µL of A Substrate, followed by 50 µL of B substrate in each well. Stir well.
8. Incubate for 15 minutes at 37ºC ± 1ºC. Times are referred to the first addition. A blue colour will be developed.
9. Transfer using a pipette 50 µL of blocking solution in all the wells according to the same timing and order as for TMB addition. Stir well. The blue colour turns on yellow.
10. Within 30 minutes read the absorbance (A) values for each well at 450 nm wavelength with a 600-650 nm reference filter.

Note: if a double wavelength spectrophotometer is available, set up the reference filter between 600-650 nm. Microwells readings at 450 nm without reference filter can result in higher absorbance values due to background.

### WASHING PROCEDURE

Wash efficiently in order to remove completely sample or various components, it is a critical requirement for ELISA procedure.

### A. Automated washing

1. Aspirate completely from all the wells.
2. Fill up all the wells till the edge (300 µL) during the washing cycle.
3. At the end of the five (5) washings, invert the plate and contact strongly with absorbent paper in order to assure that all the washing buffer is removed.
4. The automated plate must be maintained efficient in order to guarantee the washing. The cleaning instructions must be executed in every time.

### B. Manual washing

1. Discard the plate content into appropriate waste containers.
2. Fill up the wells with washing buffer using a suitable bottle. Avoid the washing buffer to be bubbled because that can reduce the washing efficiency. Immediately eliminate the washing buffer from the wells.
3. Fill up the wells with the washing buffer and immediately discard.
4. Repeat step (3) three times. This will make a total of five (5) washings with the washing buffer.
5. After the last washing, eliminate the content of the wells and contact strongly with absorbent paper in order to assure that all the washing buffer is removed

### RESULTS

83 negative controls and 22 idiopathic sterility affected women have been analysed; OD (Optical Density) values are reported below. Particularly average values (standard deviation) are equal to 0.132 (SD 0,011) and 0,260 (SD 0,042) for control and patient groups, respectively. Thus OR two group difference results to be 0.13 with 95 % confidence interval from 0.07 to 0.19.

Figure 6 shows the optical density distribution of analysed cases and controls.

The two group comparison according to Mann-Whitney non parametric test pointed out a statistically remarkable difference of antibody levels (p 0,0016).

The analysis according to ROC curve results in 0.74 area (95 % Cl from 0.60 to 0.87).

The results demonstrate that the extent of antibody response is remarkable higher for analysed sterile patients than controls.

### Example 2: Study on the ability to elicit an immune response in mouse by PLAC1 peptide administration.

The study has been carried out on murine experimental model (fertile) in order to demonstrate that it is possible an immune antibody response to be elicited (namely measuring anti-PLAC1 antibody response) and that this determines in vivo biological consequences, whether as result from interference in earlier stages of embryo implant with consequent sterility, or interference with the normal pregnancy evolution with consequent abortion.

To this end a mixture of synthetic peptides extracted from PLAC1 protein sequence and Keyhole Limpet Hemocyanin (KLH) carrier molecule conjugated as extensively described in literature (e.g. Cancer Immunology, Immunotherapy 2005. Volume 54, Number 5, 424-430) has been used as antigen.

In particular the philogenetically conserved human and murine shared PLAC1 region has been selected. The identified region is from D 33 to P 127aminoacids.

Below human and murine sequences are reported:
**HUMAN PLAC1 SEQUENCE (SEQ ID No: 1)**
>sp|Q9HBJ0|PLAC1_HUMAN Placenta-specific protein 1
**MURINE PLAC1 SEQUENCE (SEQ ID NO: 4)**
>sp|Q9JI83|PLAC1_MOUSE Placenta-specific protein 1

Antigenicity analysis using algorithms (e.g. Welling G.W., Weijer W.J., Van der Zee R., Welling-Wester S. FEBS Lett. 188:215 - 218; Kolaskar AS, Tongaonkar PC. FEBS Lett.276: 172-4) suggests that from the 61 to 128 aminoacids provide possibly good antigenic determinants.

For immunization procedures, therefore, the following peptides have been synthesized and used:
**Plac1 Pep (61-79 aa)**
   CPPNHVQPHAYQFTYRVTE (SEQ ID NO: 5)
**Plac1 Pep (80-96 aa)**
   CGIRAKAVSQDMVIYST (SEQ ID NO: 6)
**Plac1 Pep (97-115 aa)**
   EIHYSSKGTPSKFVIPVSC (SEQ ID NO: 7)
**Plac1 Pep (116-128 aa)**
   AAPQKSPWLTKPC (SEQ ID NO:8)

### ANTIGEN PREPARATION

The antigen is conjugated to KLH using Sulfo-SMCC Sulfosuccinimidyl-4-(N-maleimidomethyl)cyclohexane-1-carboxylate). In fact, SMCC and its analogous water soluble Sulfo-SMCC are molecules named "crosslinkers" because suitable to conjugate molecules of interest (Brinkley, M.A. (1992) Bioconjugate Chem. 3:2-13. ; Mattson, G., et al. (1993) Molecular Biology Reports 17:167-83.) Then antigen is aliquoted in amounts as needed for various immunization times as below specified. Each immunization will be carried out with:
T0, 15 ug of each peptide + Complete Freund's Adjuvant (CFA);
T21, T28, T35, 15 ug of each peptide + Incomplete Freund's Adjuvant (IFA).

Preservation of Aliquots at -20°C.

### Booster preparation

At time of use, antigen aliquot is thawn and to the initial volume an equal volume of CFA (Complete Freund' s Adjuvant) is added for first booster, or IFA (Incomplete Freund's Adjuvant) for remaining boosters. Antigen and the adjuvant are mixed many times in a 5 ml syringe to obtain an opalescent and homogenous emulsion without phase separations.

After emulsion homogeneity is assured, the inoculation of 60 ug/mouse total amount of peptide mix in 80 µl of Ag+ Adjuvant emulsion.
**Administration route:** Subcutaneous, neck area
**Strain:** Balb/c from Charles River Laboratories Italy s.r.l.
**Number of animals under study:** 18 (12 immunized with the peptide mix + 6 immunized with an equal volume of PBS + Adjuvant)
**Animal number/cage at time of the immunization** (6)
**Age/weight at study start:** Lot: 13/04/11
   **Sex:** Female
**Quarantine and acclimatization time:** 1 week.

**Sanitary condition:** The animals will arrive from SPF (Specific Pathogen Free) breeding.

**Housing:** SPF conditions

**The immunization schedule involves the following steps:**
**Day 0 (T0). Immunization start** Before antigen inoculation 100 µl of blood will be draw from each animal, in order to obtain an pre-immune serum sample of approximately 50 µl that will constitute negative control at time of the determination of the titre for immune serum. A clear, impurity free, straw-yellow serum will be obtained. Then the antigen inoculation will be carried out (first booster).
**Day 21 (T21.) First booster.** Inoculation of second booster 21 days after first immunization.
**Day 28 (T28). Second booster** Inoculation of third booster 7 days after second immunization and a 100 µl blood withdrawal will be carried out to obtain 50 µl of immune serum to be tested using ELISA method.

Thus in total there are immunized:
12 female mouse inoculated with peptide mixture;
6 female mouse as negative control inoculated with PBS.

### Results:

The experiment shows that we are able to induce a significant antibody response against PLAC1 peptides in mice after immunization.

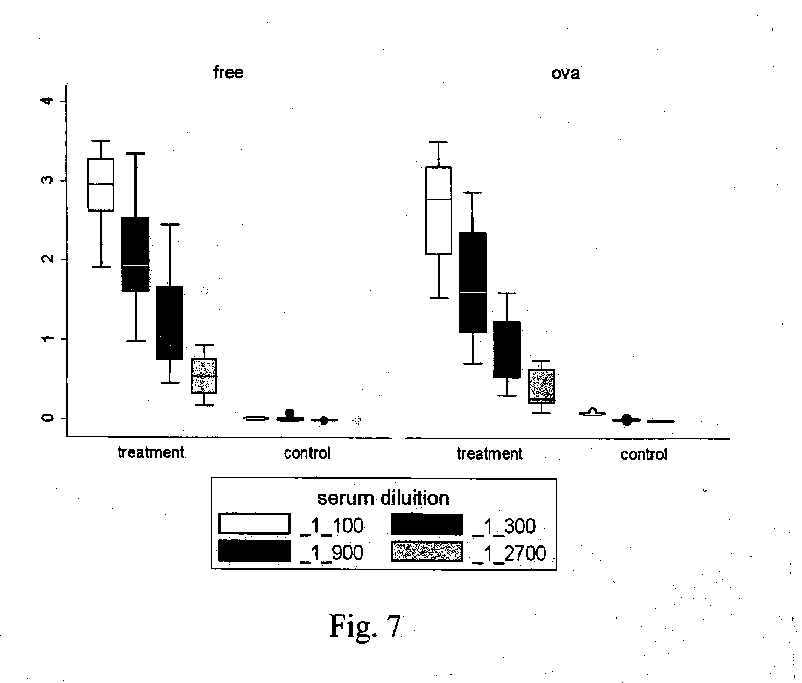

In figure 7 average O.D(optical density) values and related standard deviation obtained with ELISA method at various dilutions are reported. According to these results the antibody presence against peptides used for the immunization procedure is demonstrated, both when used as such (free) and ovalbumin conjugated in order to coat Elisa plate (ova).

The T-Student test is used for the comparison of 12 immunized mouse and 6 negative controls (immunized with PBS and adjuvant). For each dilution the test resulted significant with p=0.0016 for highest dilution (1: 2700).

Also the non parametric Mann-Whitney test (Two-sample Wilcoxon rank-sum-Mann-Whitney- test) resulted significant establishing that the antibody titre is higher for immunized group than controls at all tested dilutions (p= 0,0006 for highest dilution).

Therefore, the result of the study show that it is possible to develop an important antibody response against PLAC1 peptides following the administration of protein or portions thereof. It is therefore reasonable to argue that it is possible to induce an active immune response against PLAC1 and that this could be used for post-coital contraceptive end.

### Example 3: Study on immunogenicity of PLAC1 peptides

In addition to 4 peptides used for the immunization of mouse in example 2, 3 peptides belonging to a Plac1 region, with not high murine homology, extremely immunogenic (aa 126-155) as result of calculations carried out according to Welling et al., have been analysed.

Said 3 designed peptides, everyone of 12 aminoacids, are overlapped for 3 aa as below shown:
Pep. (126-137) KPCSMRVASKSR (SEQ ID NO:9)
Pep. (135-146) KSRATAQKDEKC (SEQ ID NO:10)
Pep. (144-155) EKCYEVFSLSQS (SEQ ID NO:11)

Human sera are tested for reactivity against total peptide pool. Sera (each tested in duplicated) from two subjects previously recognized as positive for anti-PLAC1 antibodies (full length) at dilution 1:20 have been tested.

Said sera resulted positive (compared to irrelevant peptides derived from other sequences and used as negative controls or single "blank" secondary antibody) when tested against protein regions considered immunogenic by us (peptides).

In particular the two tailed T-Student test resulted significant p= 0.004.

Conclusion: peptides identified as possibly immunogenic are in vivo recognized in women and can, all or some thereof, be used in test for identification of reactivity against PLAC1. Table 3 shows Elisa values.

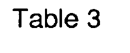

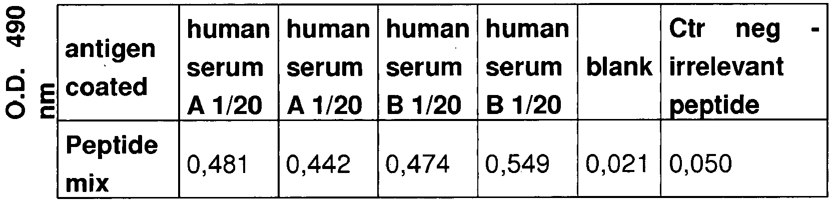

## Claims

1. Use of antibodies against PLAC1 protein as biomarkers of infertility for women and animal females expressing said protein.

2. Use according to claim 1 wherein the infertility is idiopathic.

3. Use according to claim 1 wherein the animals are selected from the group consisting of primate, bovine, equine, swine, mice, rat.

4. Method for *in vitro* diagnosis of infertility for women and animal females expressing said protein, by detection of anti PLAC1 immune response, said immune response being selected from cell-mediated or antibody response.

5. Method according to claim 4, wherein the infertility is idiopathic.

6. Method according to claim 4, wherein the animals are selected from the group consisting of primate, bovine, equine, swine, mice, rat.

## Patentansprüche

1. Verwendung von Antikörpern gegen das PLAC1-Protein als Biomarker der Unfruchtbarkeit für Frauen und weibliche Tiere, welche dieses Protein exprimieren.

2. Verwendung nach Anspruch 1, wobei die Unfruchtbarkeit idiopathisch ist.

3. Verwendung nach Anspruch 1, wobei die Tiere aus der Gruppe, die aus Primaten, Rindern, Pferden, Schweinen, Mäusen und Ratten besteht, ausgewählt sind.

4. Verfahren zur *in vitro* Diagnose von Unfruchtbarkeit für Frauen und weibliche Tiere, welche das betreffende Protein exprimieren, durch Nachweis einer Anti-PLAC1-Immunantwort, wobei die Immunantwort aus zellvermittelter Antwort oder Antikörperantwort ausgewählt ist.

5. Verfahren nach Anspruch 4, wobei die Unfruchtbarkeit idiopathisch ist.

6. Verfahren nach Anspruch 4, wobei die Tiere aus der Gruppe, die aus Primaten, Rindern, Pferden, Schweinen, Mäusen und Ratten besteht, ausgewählt sind.

## Revendications

1. Utilisation d'anticorps dirigés contre la protéine PLAC1 comme biomarqueurs de la stérilité chez la femme et les femelles animales exprimant ladite protéine.

2. Utilisation selon la revendication 1, où la stérilité est idiopathique.

3. Utilisation selon la revendication 1, où les animaux sont choisis dans le groupe constitué par les primates, le boeuf, le cheval, le porc, la souris, le rat.

4. Procédé de diagnostic in vitro de la stérilité chez la femme et les femelles animales exprimant ladite protéine, par détection d'une réponse immunitaire anti-PLAC1, ladite réponse immunitaire étant choisie parmi la réponse à médiation cellulaire ou la réponse d'anticorps.

5. Procédé selon la revendication 4, dans lequel la stérilité est idiopathique.

6. Procédé selon la revendication 4, dans lequel les animaux sont choisis dans le groupe constitué par les primates, le boeuf, le cheval, le porc, la souris, le rat.


### SEQUENCE LISTING

<110> Università degli Studi Di Foggia
<120> Use of anti PLAC1 protein antibodies as biomarkers of infertility, diagnostic kit for the detection of the immune response against PLAC1 and use of PLAC1 protein in therapeutic and contraceptive fields
<130> PCT28366
<150> RM2010A000386
   <151> 2010-07-14
<160> 11
<170> PatentIn version 3.5
<210> 1
   <211> 212
   <212> PRT
   <213> human PLAC1 protein
<400> 1
   Met Lys Val Phe Lys Phe Ile Gly Leu Met Ile Leu Leu Thr Ser Ala
   1               5                   10                  15
   Phe Ser Ala Gly Ser Gly Gln Ser Pro Met Thr Val Leu Cys Ser Ile
               20                  25                  30
   Asp Trp Phe Met Val Thr Val His Pro Phe Met Leu Asn Asn Asp Val
           35                  40                  45
   Cys Val His Phe His Glu Leu His Leu Gly Leu Gly Cys Pro Pro Asn
       50                  55                  60
   His Val Gln Pro His Ala Tyr Gln Phe Thr Tyr Arg Val Thr Glu Cys
   65                  70                  75                  80
   Gly Ile Arg Ala Lys Ala Val Ser Gln Asp Met Val Ile Tyr Ser Thr
                   85                  90                  95
   Glu Ile His Tyr Ser Ser Lys Gly Thr Pro Ser Lys Phe Val Ile Pro
               100                 105                 110
   Val Ser Cys Ala Ala Pro Gln Lys Ser Pro Trp Leu Thr Lys Pro Cys
           115                 120                 125
   Ser Met Arg Val Ala Ser Lys Ser Arg Ala Thr Ala Gln Lys Asp Glu
       130                 135                 140
   Lys Cys Tyr Glu Val Phe Ser Leu Ser Gln Ser Ser Gln Arg Pro Asn
   145                 150                 155                 160
   Cys Asp Cys Pro Pro Cys Val Phe Ser Glu Glu Glu His Thr Gln Val
                   165                 170                 175
   Pro Cys His Gln Ala Gly Ala Gln Glu Ala Gln Pro Leu Gln Pro Ser
               180                 185                 190
   His Phe Leu Asp Ile Ser Glu Asp Trp Ser Leu His Thr Asp Asp Met
           195                 200                 205
   Ile Gly Ser Met
       210
<210> 2
   <211> 39
   <212> DNA
   <213> artificial
<220>
   <223> forward primer for the amplification of the PLAC1 gene from nucleotide 67 to 636
<400> 2
   caccaccacg gcgtccaaag tccaatgact gtgctgtgc 39
<210> 3
   <211> 50
   <212> DNA
   <213> artificial
<220>
   <223> reverse primer for the amplification of the PLAC1 gene from nucleotide 67 to 636
<400> 3
   cgagcgaagg cgtcagatta tcacatggac ccaatcatat catctgtgtg 50
<210> 4
   <211> 173
   <212> PRT
   <213> murine PLAC1 protein
<400> 4
   Met Asn Leu Arg Lys Phe Leu Gly Gly Thr Val Leu Val Ala Phe Met
   1               5                   10                  15
   Leu Phe Ser Tyr Ser Glu Gln Asn Gln Val Asn Val Leu Cys Ser Thr
               20                  25                  30
   Asp Trp Phe Met Val Thr Val His Pro Phe Leu Leu Asn Asn Asp Val
           35                  40                  45
   Tyr Val His Phe Tyr Glu Val His Leu Gly Leu Gly Cys Pro Pro Asn
       50                  55                  60
   His Val His Pro His Phe Tyr Gln Phe His Tyr Arg Val Thr Glu Cys
   65                  70                  75                  80
   Gly Ile Arg Ile Lys Ala Val Ser Pro Asp Val Val Ile Tyr Ser Ser
                   85                  90                  95
   Glu Ile His Tyr Ala Ser Lys Gly Ser Ser Thr Lys Tyr Val Ile Pro
               100                 105                 110
   Val Ser Cys Ala Ala Pro Arg Arg Ser Pro Trp Leu Thr Lys Pro Tyr
           115                 120                 125
   Ser Ala Lys Ala Pro Ser Asn Asn Met Gly Ala Thr Pro Lys Asn Asp
       130                 135                 140
   Thr Ser Tyr His Val Phe Thr Leu Pro Glu Pro Ser Glu Gln Pro Asn
   145                 150                 155                 160
   Cys Ser Cys Pro Pro Tyr Val Tyr Asn Gln Lys Ser Met
                   165                 170
<210> 5
   <211> 19
   <212> PRT
   <213> antigenic epitope of PLAC1 protein from 61 aa to 79 aa
<400> 5
   Cys Pro Pro Asn His Val Gln Pro His Ala Tyr Gln Phe Thr Tyr Arg
   1               5                   10                  15
   Val Thr Glu
<210> 6
   <211> 17
   <212> PRT
   <213> antigenic epitope of PLAC1 protein from 80 aa to 96 aa
<400> 6
   Cys Gly Ile Arg Ala Lys Ala Val Ser Gln Asp Met Val Ile Tyr Ser
   1               5                   10                  15
   Thr
<210> 7
   <211> 19
   <212> PRT
   <213> antigenic epitope of PLAC1 protein from 97aa to 115aa
<400> 7
   Glu Ile His Tyr Ser Ser Lys Gly Thr Pro Ser Lys Phe Val Ile Pro
   1               5                   10                  15
   Val Ser Cys
<210> 8
   <211> 13
   <212> PRT
   <213> antigenic epitope of PLAC1 protein from 116aa to 128aa
<400> 8
   Ala Ala Pro Gln Lys Ser Pro Trp Leu Thr Lys Pro Cys
   1               5                   10
<210> 9
   <211> 12
   <212> PRT
   <213> antigenic epitope of PLAC1 human protein from 126 aa to 137 aa
<400> 9
   Lys Pro Cys Ser Met Arg Val Ala Ser Lys Ser Arg
   1               5                   10
<210> 10
   <211> 12
   <212> PRT
   <213> antigenic epitope of PLAC1 human protein from 135aa to 146aa
<400> 10
   Lys Ser Arg Ala Thr Ala Gln Lys Asp Glu Lys Cys
   1               5                   10
<210> 11
   <211> 12
   <212> PRT
   <213> antigenic epitope of PLAC1 human protein from 144aa to 155aa
<400> 11
   Glu Lys Cys Tyr Glu Val Phe Ser Leu Ser Gln Ser
   1               5                   10